# EUROPEAN PATENT APPLICATION

(11) **EP 4 427 782 A1**
(43) Date of publication of application: **11.09.2024**
(21) Application number: 23160528.8
(22) Date of filing: 07.03.2023
(51) Int. Cl.: A61M 16/04, C08G 18/00

(54) **AN AIRWAY TUBE DEVICE**

(71) Applicant: The Provost, Fellows, Scholars and other Members of Board of Trinity College Dublin, Dublin 2 (IE)
(72) Inventor: Martin-Loeches, Ignatio, Dublin, D02 (IE); Naylor, Emily, Dublin, D18 (IE); Tejada-Rios, Beatriz, 06003 Badajoz (ES); Ruiz-Hernandez, Eduardo, Dublin, D02 (IE)
(74) Representative: Purdylucey Intellectual Property

(57) **Abstract**

An airway tube device (1, 30) comprises an airway tube (5) having a distal end (6), a proximal end (7), an internal surface (9) defining a lumen (8) of the tube, and an outer surface, a removable thin-film tubular liner (15) having an inner lumen (19) attached to the inner surface (9) of the airway tube (5), and, optionally, a lanyard (20) extending through the inner lumen (19) of the removable thin-film tubular liner (15) and having a distal end (21) attached to a distal end (17) of the thin-film tubular liner (15) and a proximal end (16) extending from a proximal end (7) of the airway tube. The device is pre-formed, and the removable thin-film tubular liner (15) comprises a thin film coating covalently bonded to the internal surface (9) of the airway tube (5) by a coating technique.

## Description

### Field of the Invention

The present invention relates to an airway tube device, in particular an endotracheal tube (ETT) device, and uses thereof to invasively ventilate a patient.

### Background to the Invention

Intubation is a procedure that can help save a life when someone can't breathe by him/herself. With the use of a tool called laryngoscope, an endotracheal tube (ETT) is guided into the mouth or nose then trachea. The tube keeps the airway open so air can get to the lungs. Intubation is usually performed in a hospital during an emergency or before surgery. There are many reason for the need of the use of ETT : Airway obstruction, injury or trauma to your neck, abdomen or chest that affects the airway, loss or low level of consciousness, need for surgery that will make you unable to breathe on patient's own, acute respiratory (breathing) failure or apnoea (a temporary stop in breathing) and risk for aspiration. The endotracheal tube is attached to a mechanical ventilator to assist the patient's breathing. An ETT can remain in place to provide ventilator-assisted breathing for days or weeks.

Many complications of endotracheal tube intubation can occur during placement or shortly after the endotracheal tube is removed, including bleeding, injury to the larynx or trachea, lack of oxygen and partial or full collapse of the lung. One of the most serious and prevalent problems associated with use of ETT's is the development of a lower respiratory tract infection when the patient is on a ventilator, so called Ventilator Associated Pneumonia (VAP). VAP is a common and serious nosocomial infection in mechanically ventilated patients and results in high mortality, prolonged intensive care unit- (ICU) and hospital-length of stay and increased costs. ET contributes substantially to the pathogenies of pneumonia. As there is growing evidence of medical device-related infections and biofilms. ET biofilm formation is an early and constant process in intubated patients. The ET provides an ideal opportunity for bacterial and fungal adhesion and biofilm formation on its inner luminal surface. ET surface is a nest for bacterial biofilm formation.

US2010199448 and US20170065367 describe devices for the cleaning of an endotracheal tube while a patient is being supported by a ventilator connected to the endotracheal tube, the devices operating on the basis of a mechanically actuated non-inflatable cleaning device for scraping debris (e.g., biofilm) from an interior wall of an endotracheal tube. There are potential life threatening major adverse events such as partial or complete ETT occlusion, leading to increased airway resistance and work of breathing, desaturation, tachycardia, hypotension, ETT displacement requiring reintubation, tracheal injury evidenced by suctioning of bloodstained secretions, pneumothorax, need of ventilatory settings change after manoeuvre.

US2015/0250966 describes an inner liner for an ETT and a method of deploying the inner liner into the ETT and subsequent removal and replacement of the inner liner. The inner liner comprises a tubular sleeve of material configured to nest inside the ETT having a distal end, a proximal end, and a lanyard that extends through the sleeve and is attached to the distal end. The distal end of the sleeve includes a noose to which the lanyard is attached. An introducer device is required to introduce the sleeve of material into the ETT, and the sleeve of material may be formed from a material that non-chemically couples with the inner surface of the ETT by electrostatic or Van der Walls forces. Removal of the sleeve is achieved by retracting the lanyard through the lumen of the inner sleeve to fold the inner sleeve inwardly and retract it proximally until it is fully removed from the ETT. Once this is completed, a fresh inner sleeve is then introduced into the ETT.

A number of problems are associated with this system. The system requires that an inner sleeve is introduced into the device immediately prior to use, which is problematical in the case of use in an emergency with trauma patients. In addition, the requirement that the inner sleeve does not chemically bond with the inner surface of the ETT results in the inner sleeve being weakly or incompletely adhered to the inner surface of the ETT. When this occurs at the distal end of the ETT, gaps between the ETT and inner sleeve will result, providing gaps for aspirations to access and adhere to the inner surface of the ETT, resulting in focal points for biofilm formation. In these circumstances, removal of the inner sleeve will be insufficient in preventing biofilm formation and resultant Ventilator Assisted Pneumonia.

It is an object of the invention to overcome at least one of the above-referenced problems.

### Summary of the Invention

The Applicant has addressed the problems of the prior art by providing a pre-formed endotracheal tube (ETT) device having a removable inner liner that is covalently bonded to the inner surface of the ETT, and a lanyard that is attached to a distal end of the inner liner and can be pulled proximally to peel the inner liner inwardly and away from the inner surface and remove the inner liner. The preforming of the multi-lumen device allows the inner liner to be covalently bonded to the inner surface of the ETT, and the choice of the materials may be tuned to determine the bonding strength of the inner liner and ETT to ensure close adherence of the two lumens while allowing the inner liner to be removed. Importantly, the covalent bonding between the two lumens ensures that aspirations cannot access the inner surface of the ETT when a patient is intubated. The device may be used out of the pack to intubate a patient, and, in the case of prolonged intubation, the inner liner may be removed after 48 hours thereby removing any biofilm formed on the inner liner, and the ETT without the inner liner may be left *in-situ* then removed without the risk of VAP. The device is easy to use and does not require any specialist introducer equipment for use in patients that are intubated for more than 48 hours. The material of the inner liner may be configured to adhesively bond to the material of the ETT. For example, the inner liner may comprise a polymer functionalised with chemical moieties configured to covalently bond with functional groups in the ETT.

In a first aspect, the invention provides an airway tube device comprising:
an airway tube having a distal end, a proximal end, an inner surface defining a lumen of the tube, and an outer surface;
a first removable tubular liner having an inner lumen and disposed in at least a distal part of the airway tube; and
optionally, a first lanyard extending through the inner lumen of the first removable tubular liner and having a distal end attached to a distal end of the first removable tubular liner and a proximal end extending from a proximal end of the airway tube,
characterised in that the first removable tubular liner is a thin-film coating disposed on, and covalently bonded to, the inner surface of the airway tube.

In any embodiment, the airway tube is an endotracheal tube (ETT).

In any embodiment, the airway tube is formed from PVC, rubber or silicone.

In any embodiment, the airway tube has a diameter of from 2 to 12 mm in diameter.

The airway tube may contain more than one lumen, in which case at least one of the lumens comprises the first removable tubular liner.

In any embodiment, the removable inner tube comprises a polymer material comprising first functional groups configured to crosslink with second functional groups in the endotracheal tube.

In any embodiment, the second functional groups are plasticiser moieties and the first functional groups are configured to crosslink with the plasticiser moieties.

In any embodiment, the first functional groups are catechol moieties and in which the second functional groups are ester-derived plasticisers.

In any embodiment, the removable inner tube is a coated tube.

In any embodiment, the device comprises at least two lanyards extending through the inner lumen of the removable inner tube, each having a distal end attached to a distal end of the removable inner tube.

In any embodiment, the ETT has an outer diameter of 5-15 mm or 6-13 mm. In any embodiment, the ETT has an internal diameter 4 to 12 mm or 5 to 10 mm. In any embodiment, the ETT has a wall thickness of 1.5 to 3.5 mm.

The invention also relates to an airway tube device according to the invention packaged in a sterile packaging.

In any embodiment, the device comprises a second removable tubular liner coated on, and covalently bonded, to the (first) removable tubular liner. This allows the device to be used for prolonged periods of intubation, where the second removable tubular liner is removed after a first intubation period of, for example, up to 40 or 36 hours, and the (first) inner removable inner tube is removed after a second intubation period of, for example, up to 40 or 36 hours. The device may comprise second, third or more removable tubular liner.

In another aspect, the invention provides a method of manufacturing an airway tube device, comprising the steps of:
forming an airway tube having a distal end, a proximal end, an inner surface defining a lumen of the tube, and an outer surface; and
forming a removable tubular liner comprising a thin film coating on the inner surface of the endotracheal tube in which the thin film coating is covalently bonded to the inner surface of the airway tube.

In any embodiment, the method comprises attaching a distal end of a lanyard a distal end of the removable tubular liner, in which a proximal end of the lanyard extends from a proximal end of the airway tube.

In any embodiment, the airway tube is an endotracheal tube (ETT).

In any embodiment, the removable tubular liner consists of a thin film coating.

In any embodiment, the removable tubular liner is formed on the inner surface of the airway tube by (liquid) dip coating.

In any embodiment, the removable tubular liner is formed on the inner surface of the airway tube by casting.

In any embodiment, the method comprises attaching a distal end of at least two lanyards to a distal end of the removable tubular liner, in which a proximal end of each lanyard extends from a proximal end of the endotracheal tube.

In some embodiments, a lanyard is not required. In these embodiments, the removable tubular liner comprises a proximal end that extends out of the distal end of the airway tube. In use, a user can grip the proximal end of the tubular liner and pull in out of the airway tube.

In any embodiment, the removable tubular liner comprises a polymer material comprising first functional groups configured to crosslink with second functional groups in the endotracheal tube.

In any embodiment, the second functional groups are plasticiser moieties and the first functional groups are configured to crosslink with the plasticiser moieties.

In any embodiment, the first functional groups are catechol moieties and in which the second functional groups are ester-derived plasticisers.

In any embodiment, the method comprises forming a second removable tubular liner comprising a thin film coating on an inner surface of the (first) removable tubular liner in which the second removable tubular liner is covalently bonded to the inner surface of the first removable tubular liner. The second removable tubular liner may be formed on an inside surface of the (first) removable tubular liner by coating, for example dip coating.

In another aspect, the invention provides a method of intubation comprising the steps of:
inserting an endotracheal tube device according to the invention into an airway of a subject;
ventilating the subject using the endotracheal tube device during a first ventilation period;
while the endotracheal tube device is *in-situ* in the subject's airway, removing the removable tubular liner (e.g. by retracting the proximal end of the or each lanyard or pulling a proximal end of the liner) and leaving the endotracheal tube *in-situ* in the subject's airway;
ventilating the subject using the endotracheal tube during a second ventilation period; and
removing the endotracheal tube from the subject's airway at the end of the second ventilation interval.

In any embodiment, the first ventilation period is at least 48, 72, or 96 hours.

In any embodiment, the second ventilation interval lasts until the endotracheal tube device is removed from the subject, for example at least 1, 2 or 3 weeks.

In any embodiment, the device comprises first and second removable tubular liner, in which the method comprises:
inserting the endotracheal tube device into an airway of a subject;
ventilating the subject using the endotracheal tube device during a first ventilation period;
while the endotracheal tube device is in-situ in the subject's airway, removing the second removable tubular liner leaving the endotracheal tube in-situ in the subject's airway;
ventilating the subject using the endotracheal tube during a second ventilation period;
while the endotracheal tube device is in-situ in the subject's airway, removing the (first) removable tubular liner leaving the endotracheal tube in-situ in the subject's airway;
ventilating the subject using the endotracheal tube during a third ventilation period; and
removing the endotracheal tube from the subject's airway at the end of the third ventilation interval.

Other aspects and preferred embodiments of the invention are defined and described in the other claims set out below.

### Brief Description of the Figures

**Figure 1** is a side elevational view of an airway device according to a first embodiment of the invention.
**Figure 2** is a cross-sectional view through the device taken along the lines II-II of Figure 1.
**Figure 3** is detailed, partly cut-away view of a section of the device of Figure 1
**Figures 4A, 4B and 4C** are a cross sectional views taken along the lines IV-IV of Figure 2 of a distal end of the airway device during withdrawal of the thin-film tubular liner.
**Figure 5** is a perspective view of a device according to a second embodiment of the invention.
**Figure 6** is a perspective view of the device of Figure 2 after withdrawal of the thin-film tubular liner.
**Figure 7** is a block diagram illustrating one method of manufacturing the airway tube device of the invention.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "airway tube" refers to a hollow tube suitable for advancement into a subject's thorax to provide air to the subject's lungs. The tube is usually polymeric, for example rubber, PVC or silicone, and is configured for advancement through the subject's mouth. A proximal end of the airway tube is generally configured for attachment to a manual or automatic ventilator device. A distal end of the tube may comprise an annular inflatable balloon that can be inflated once it has passed a subject's larynx. Examples of airway tubes include endotracheal tubes, nasopharyngeal tubes and a tracheostomy tube. Airway tubes typically have a diameter of from 2 to 12 mm in diameter. Typically, the airway tube comprises a plasticiser, especially an ester-based plasticiser. Ester-based plasticisers, and methods of manufacture thereof, are described in WO2014182083A1 and US20030061969.

As used herein, the term "removable tubular liner" refers to a thin film coating that is applied and covalently bonded to an inside of the airway tube along at least a distal end of the airway tube and preferably along substantially all of the airway tube. The coating may be applied by dip-coating the inside of the airway tube. The liner is typically a thin-film liner, meaning it has a wall thickness of 0.2 mm to 1.0 mm. The liner is pre-formed on the airway tube, and can be easily peeled away from the surface of the airway tube by pulling on one or more lanyards attached to a distal end of the liner, or in another embodiment, by pulling a proximal end of the liner that extends proud of a proximal end of the airway tube. In any embodiment the thin film coating comprises a polymer (polyester, polyamide, polyacrylate, polyethylene glycol, polyoxetane, polystyrene, polypeptide, polysaccharide), preferably a bacteriostatic polymer such as hyaluronic acid (HA). Typically, the HA has a MW of > 1MDa, for example 1-10 MDa, 1-5 MDa, or 1-2 MDa. The bonding strength between the liner and airway tube will be sufficient to keep the liner closely adhered to an inside surface of the airway tube during use but allow the liner to be peeled away from the airway tube when the lanyard is pulled by a user. The bonding strength can be tuned by choosing specific materials for the liner and the airway tube such that the liner and airway tube adhesively bind when the liner is formed on the airway tube (generally by coating technique). The device may include second or more removable tubular liners, where the liner is coated on and covalently bonded to the inner surface of a previously formed inner liner. Thin film coatings and method of application thereof (including methods of dip coating) on to surfaces are described in:
- Butt et al. (Thin-Film Coating Methods: A Successful Marriage of High-Quality and Cost-Effectiveness-A Brief Exploration. Coatings 2022, 12, 1115);
- Zamboni et al, (Carbohydrate Polymers 2020, 260, 117803); and
- Romano et al, (J Bone Jt Infect 2017, 2(1), 63).

As used herein, the term "thin film" as applied to the removable tubular liner means a liner having a thickness of less than 1.0 mm and typically 0.2 to 1.0 mm, 0.2 to 0.7 mm, 0.2 to 0.5 mm.

As used herein, the term "lanyard" refers to a suture or thin wire that extends through the inner lumen of the device and attaches to a distal end of a removable tubular liner. A proximal end of the lanyard extends from a proximal end of the device. Pulling the lanyard causes the distal end of the tubular liner to detach from the distal end of the airway tube and fold in on itself as it is being detached and removed from the airway tube. Typically, each removable tubular liner has two associated lanyards, which are generally attached to opposed sides of the distal end of the tubular liner. The proximal end of the lanyard may include a tab element to facilitate holding the end of the lanyard.

As used herein, the term "dip coating" is a simple and effective technique which is commonly used in manufacturing across a wide range of different industries. Within research and development, it has become an important coating method for the fabrication of thin films using a purpose-built dip coater. When the process is optimised, dip coating can be used to produce highly uniform films. Importantly, key factors such as film thickness can be easily controlled. Method of dip coating are described in US Patent No.8313760 and Puetz et al, (Thin Solid Films 2003, 442, 53). In any embodiment, the airway tube device (airway tube and thin-film coating) is made by a method of comprising simultaneously extruding and coating the airway tube. This method is described in US6447835.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

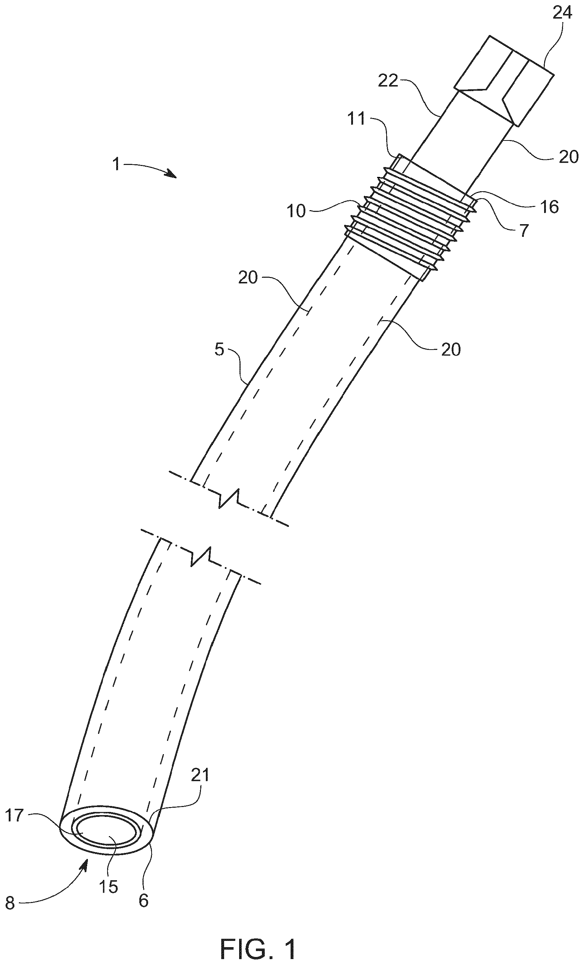

Referring to the drawings and initially Figures 1 to 3, there is illustrated an endotracheal tube (ETT) device according to the invention, indicated generally by the reference numeral 1. Only the distal and proximal sections of the ETT device are illustrated. The ETT device comprises a PVC tube 5 with an open distal end 6, an open proximal end 7, and an internal lumen 8 defined by an internal surface 9 of the PVC tube 5. The PVC tube is formed from PVC and ester-derived plasticisers. In this embodiment, the PVC tube has a length of 15 cm, a diameter of 10 mm, and a wall thickness of 2 mm. These dimensions may be varied according to the patient being treated; for example a younger patient may require a tube having a smaller diameter such as 2 to 5 mm.

The proximal end of the device comprises a moulded polymer fitting 10 comprising threads 11. The purpose of this fitting is to fluidically connect the tube with a ventilator (not shown).

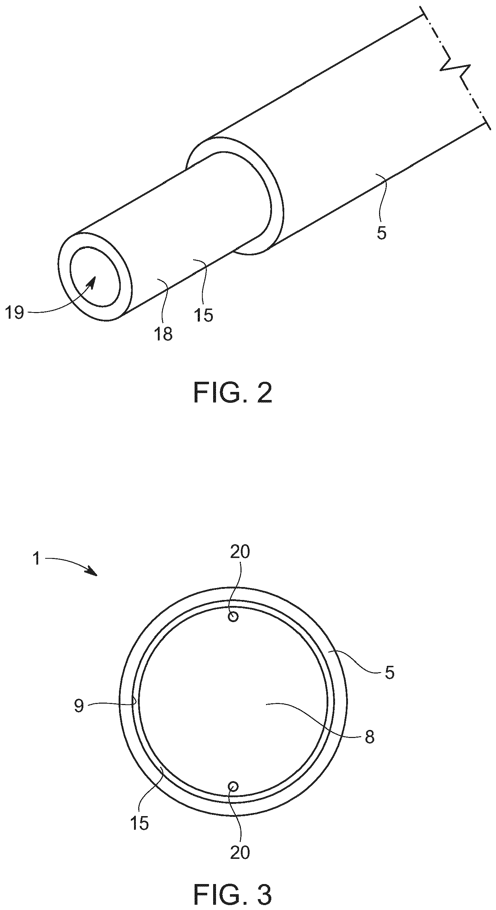

The ETT device comprises a thin-film tubular liner 15 that is coated onto the internal surface 9 along length of the PVC tube. The tubular liner has a proximal end 16, distal end 17 and sidewall 18 defining an inner lumen 19. In this embodiment, the tubular liner 15 has a wall thickness of 0.5 mm and is formed from hyaluronic acid having a MW of > 1MDa. The surface chemistry of the hyaluronic acid is modified with catechol moieties using carbodiimide crosslinker chemistry (adhesion strength to be controlled by mol% catechol) to provide adhesive bonding to the ester-derived plasticizers in the PVC tube 5. Thus, the tubular liner 15 is adhesively and covalently bonded to the internal surface 9 of the PVC tube 5. Figure 3 shows a section of the device 1 with the PVC tube 5 partly cut-away to show the thin-film tubular liner 15.

The ETT device 1 comprises two lanyards 20 (indicated with a broken line in Figure 1), each comprising a suture having a distal end 21 attached to opposite sides of the distal end 17 of the tubular liner 15 and a proximal end 22 that extends proud of the proximal end of the device 1. A grip 24 is attached to each proximal end 22 to allow a user grip and articulate the lanyards during use.

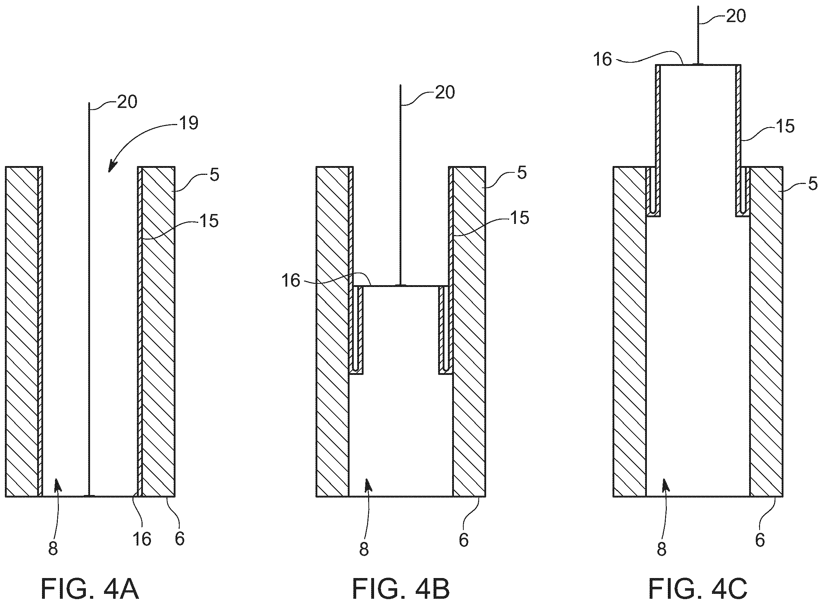

The adhesion strength of the HA liner to the internal surface of the PVC tube can be controlled by the mol% catechol in the liner such that the liner remains securely attached to the PVC tube during storage and use in a patient, and can be detached from the PVC tube by pulling the lanyards distally. Figures 4A to 4C illustrate a distal section of the device 1 during the initial stages of removal of the tubular liner 15. As can be seen from these figures, the distal end of the liner detaches from the distal end of the PVC tube when the lanyards are pulled distally and is folded inwardly on itself as it is withdrawn. This ensures that any biofilm containing surface of the liner is folded inwardly and does not come into contact with the PVC tube during retraction.

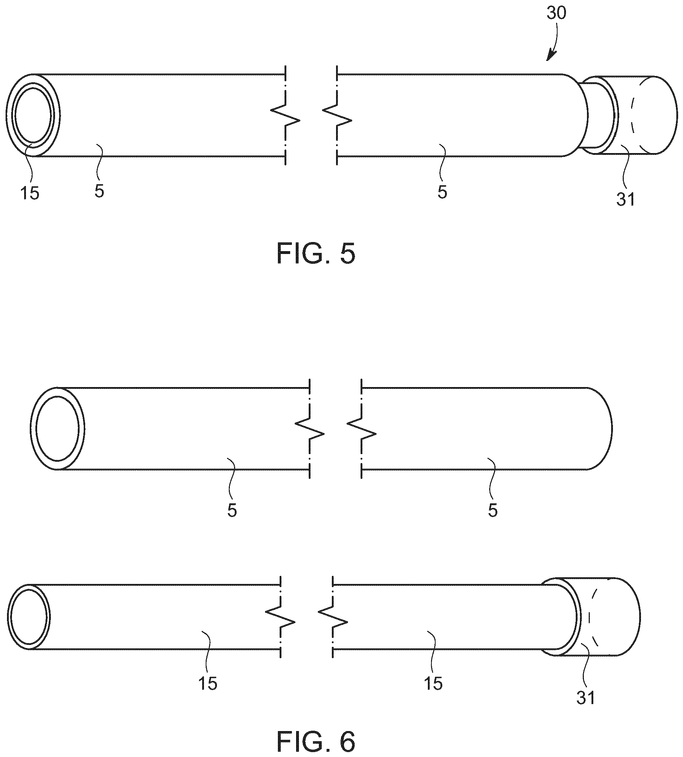

Referring to Figures 5 and 6, a further embodiment of the invention is illustrated in which parts described with reference to the previous embodiments are assigned the same reference numerals. In this embodiment, which is indicated generally by the reference numeral 30, the same PVC tube 5 and thin-film tubular liner 15 are employed, and instead of employing lanyards to detach and withdraw the liner 15 by pulling on the distal end of the liner, the liner 15 has a proximal section 31 that extends proximally of the proximal end of the PVC tube, which functions as grip for detaching and retracting the liner from the PVC tube. In this embodiment, the liner 15 has a wall thickness of 0.8mm.

In use, the ETT devices 1, 30 are removed from their packaging, and inserted into a patients mouth to establish a clear airway into the patient's lungs. Once the patient is intubated, the distal end 6 of the PVC tube may be attached to a manual ventilator or automatic ventilator and the patient may be ventilated. In the case of prolonged periods of ventilation, bacterial biofilm can form along an inner surface of the device due to aspirations from the patient. If the patient is intubated for 48 hours or more, it is likely that these biofilms will cause ventilator assisted pneumonia (VAP) in the patient. With the device of the invention, after a first period of ventilation of say 24 to 36 hours, any biofilm that has developed will be located on an inner surface of the tubular liner 15. To remove the liner, a healthcare worker detaches the ventilator from the device while the patient remains intubated with the device, and then detaches and retracts the thin film liner 15 from the PVC tube. In the device 1, this is achieved by pulling the lanyards 20 proximally until the tubular liner is fully removed from the PVC tube, whereas with the device 30, this is achieved by pulling the proximal section 31 of the liner 15. In both cases, the liner 15 (and biofilm formed on the liner) is removed from the device, thereby removing the cause of VAP. Once removed, the patient can continue to be ventilated with the PVC tube for a further ventilation period before the PVC tube is removed.

In some embodiments of the invention, the device may include a second thin-film liner coated on an internal surface of the first liner. In these embodiments, the same HA film may be employed as the liner, and the surface chemistry may be modified to allow the second thin-film liner adhesively bond to the first thin-film liner. The surface chemistry of the second thin-film liner may be tuned so that the adhesive bond between the first and second liner may be weaker than the adhesive bond between the first liner and PVC tube. This ensures that when the second liner is being detached and retracted, the first liner is not detached from the PVC tube. The use of this embodiment of the device allows the ETT device to be used for longer periods, for example up to four or more days, as the use involves a first period of ventilation before the second (innermost) liner is removed, a second period of ventilation before the first liner is removed, and then a third period of ventilation after the first liner has been detached from the PVC tube and withdrawn.

An important element of the invention is that the thin-film liner is pre-formed on the PVC tube and does not need to be inserted into the tube prior to use. Various methods known to the person skilled in the art may be employed to form a coating of the thin-film liner on the inside of the airway tube, for example a coating technology such as dip coating a thin film liner on the inside of the airway tube.

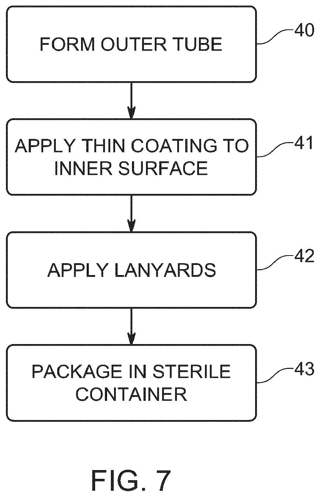

Figure 7 illustrates one embodiment of a method of making the ETT device of the invention. In a first step 40, a PVC tube with a proximal threaded section is formed by conventional injection moulding. In a second step 41, a thin-film of HA is coated onto the internal surface of the PVC tube by dip coating the inside of the PVC tube. This forms the thin-film tubular liner that adhesively binds to the inside of the PVC tube. In a third step 42, two lanyards are threaded through the lumen of the device and bonded to the distal end of the tubular liner with the proximal end of the lanyards extending from the proximal end of the PVC tube. In a fourth step 43, the device is packaged in a sterile pack.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. An airway tube device (1, 30) comprising:
an airway tube (5) having a distal end (6), a proximal end (7), an internal surface (9) defining a lumen (8) of the tube, and an outer surface;
a removable tubular liner (15) having an inner lumen (19) attached to the inner surface (9) of the airway tube (5); and
optionally, a lanyard (20) extending through the inner lumen (19) of the removable thin-film tubular liner (15) and having a distal end (21) attached to a distal end (17) of the thin-film tubular liner (15) and a proximal end (16) extending from a proximal end (7) of the airway tube,
**characterised in that** the removable tubular liner (15) comprises a thin film coating covalently bonded to the internal surface (9) of the airway tube (5).

2. An airway tube device (1, 30) according to Claim 1, in which the removable thin-film tubular liner (15) comprises a polymer material comprising first functional groups adhesively crosslinked with second functional groups in the airway tube (5).

3. An airway tube device (1, 30) according to Claim 2, in which the second functional groups are plasticiser moieties and the first functional groups are crosslinked with the plasticiser moieties.

4. An airway tube device (1, 30) according to Claim 3, in which the first functional groups are catechol moieties and in which the second functional groups are ester-derived plasticisers.

5. An airway tube device (1, 30) according to any preceding Claim, in which the removable tubular liner (15) consists of a thin film coating.

6. An airway tube device (1) according to any preceding Claim, comprising at least two lanyards (20) extending through the inner lumen (19) of the removable thin-film tubular liner (15), each having a distal end (21) attached to a distal end (17) of the removable inner tube (15).

7. An airway tube device (1) according to any preceding Claim, which is an endotracheal tube device.

8. A method of manufacturing an endotracheal tube device, comprising the steps of:
forming an endotracheal tube having a distal end, a proximal end, an inner surface defining a lumen of the tube, and an outer surface;
forming a removable inner tube comprising a thin-film coating on the inner surface of the endotracheal tube in which the thin film coating is covalently bonded to the inner surface of the endotracheal tube; and
optionally, threading a lanyard through the lumen of the endotracheal tube and attaching a distal end of the lanyard a distal end of the thin film coating, in which a proximal end of the lanyard extends from a proximal end of the endotracheal tube.

9. A method according to Claim 8, in which the removable inner tube consist of a thin-film coating that is coated on the inner surface of the endotracheal tube.

10. A method according to Claim 8 or 9, comprising threading two lanyards through the lumen of the endotracheal tube and attaching a distal end of each lanyard to a distal end of the thin film coating, in which a proximal end of each lanyard extends from a proximal end of the endotracheal tube.

11. A method according to any of Claim 8 to 10, in which the thin film coating comprises a polymer material comprising first functional groups configured to crosslink with second functional groups in the endotracheal tube.

12. A method according to Claim 11, in which the second functional groups are plasticiser moieties and the first functional groups are configured to crosslink with the plasticiser moieties.

13. A method according to Claim 12, in which in which the first functional groups are catechol moieties and in which the second functional groups are ester-derived plasticisers.

14. A method of intubation comprising the steps of:
inserting an endotracheal tube device according to any of Claims 1 to 7 into an airway of a subject;
ventilating the subject using the endotracheal tube device during a first ventilation interval;
while the endotracheal tube device is in-situ in the subject airway, removing the removable inner tube by retracting the proximal end of the or each lanyard and leaving the endotracheal tube in-situ in the subject's airway;
ventilating the subject using the endotracheal tube during a second ventilation interval; and
removing the endotracheal tube from the subjects airway at the end of the second ventilation interval.

15. A method according to Claim 14, in which the first ventilation period is up to 48 hours.
